# EUROPEAN PATENT SPECIFICATION

(11) **EP 1 868 513 B1**
(45) Date of publication and mention of the grant of the patent: **06.05.2015**
(21) Application number: 06738622.7
(22) Date of filing: 17.03.2006
(51) Int. Cl.: A61B 17/22, A61B 17/00, A61B 17/34

(54) **REMOVAL AND REPOSITIONING DEVICE**
ENTNAHME- UND REPOSITIONSVORRICHTUNG
DISPOSITIF DE RETRAIT ET DE REPOSITIONNEMENT

(30) Priority: 17.03.2005 US 663352 P; 22.12.2005 US 318083
(43) Date of publication of application: 26.12.2007
(73) Proprietor: GI Dynamics, Inc., Lexington MA 02421 (US)
(72) Inventor: LAMPORT, Ronald B., Pelham, New Hampshire 03076 (US); MELANSON, David A., Hudson, New Hampshire 03051 (US)
(74) Representative: Jenkins, Peter David
(86) International application number: PCT/US2006/009584
(87) International publication number: WO 2006/102012

(56) References cited:
- WO-A-03/073961
- US-A- 4 909 789
- US-A- 4 994 079
- US-A- 5 697 936
- US-A- 6 095 970
- US-A1- 2002 082 639
- US-A1- 2004 172 042
- US-A1- 2005 049 612
- US-B1- 6 458 074

## Description

### BACKGROUND OF THE INVENTION

Gastrointestinal implants are used for a number of treatments such as stents to treat esophageal, pyloric or colonic obstruction, and gastrointestinal liners to treat obesity or diabetes. The implants placed within the gastrointestinal tract are normally subject to substantial mechanical forces related to the digestion process. For example, peristaltic forces may force the implant to move distally. To keep the implant in place, an anchoring device is needed. Anchoring can include conventional surgical techniques, such as sutures, staples, surgical adhesives, and others. At least some anchoring devices use an interference fit, placing an implant device having a relaxed diameter larger than the diameter offered by the intestine. Other anchoring devices may include barbs that are adapted to penetrate into the surrounding muscular tissue of the gastrointestinal tract.

Often, these gastrointestinal implants, due to the complex structure of the anchoring device, may not be removed without damaging surrounding tissue, unless by resection.

US5697936 describes a device for removing an implanted elongated structure from a patient. The device includes a snare which encircles and reversibly grasps an end of the structure, and a sheath member for delivering the snare to the end of the structure to be grasped.

US2002/0082639 describes a device for deployment in a blood vessel for collecting floating debris in a filter. The device includes a collapsible proximally tapered frame for supporting the filter. The document also describes a retrieval sheath for collapsing the device for withdrawal. The retrieval sheath includes a telescoping structure including inner and outer tubular members. The inner tubular member includes a tapered flanged end.

US 6 458 074 discloses a device having the features of the preamble of claim 1.

### SUMMARY OF THE INVENTION

A first aspect of the present invention provides a device for repositioning an implantable device within a natural bodily lumen, as defined in claim 1. The device includes an inner tube defining a lumen.

The device also includes an elongated member, such as a wire having a proximal end and a distal end. The elongated member is slidably disposed within the lumen of the inner tube. In some embodiments, the elongated member is capable of a reciprocating motion. Alternatively, or in addition, the elongated member is capable of a rotational motion with respect to the inner tube.

The device also includes a grasper disposed at the distal end of the elongated member. The grasper is adapted to grasp a portion of the implantable device, such as a drawstring of a gastrointestinal liner or stent. When the drawstring is grasped and moved linearly, at least a portion of the implantable device radially collapses. The grasped device can then be repositioned within the natural bodily lumen. In some instances, the grasped device can be removed from the natural bodily lumen together with the inner tube. The grasper may be a hook or other structure that is capable of grasping a drawstring of the implantable device.

The device also includes a retrieval hood. The retrieval hood is adapted to capture at least a portion of the implantable device. For example, the retrieval hood may be advanced over a proximal portion of the device in order to facilitate removal or repositioning of the object. This is particularly advantageous when the device includes protrusions, such as barbs that might otherwise damage tissue of the natural bodily lumen. In some instances, the retrieval hood may be made of hard plastic that is transparent. The transparent retrieval hood may be advantageous to the repositioning procedure. For example, if the repositioning device is used through the working channel of an endoscope, the endoscope facilitates viewing and the transparency of the retrieval hood increases the field of view. The retrieval hood is flared to facilitate fully capturing a large anchor or stent within the hood. The retrieval hood may also be composed of flexible material, such as plastic, to minimize damage to surrounding tissue as it is introduced into the body. In some instances, the retrieval hood may include an interior ramp. The interior ramp may facilitate centering of the grasper and the inner tube within the interior of the implantable device when radially collapsing the implantable device. Alternatively, or in addition, the interior ramp may facilitate centering of the collapsed implantable device within the flared retrieval hood when the retrieval hood is advanced over the collapsed implantable device.

The device also includes an outer tube. The outer tube also defines a lumen within which the inner tube is slidably engaged. The retrieval hood is attached to the distal end of the outer tube facilitating the acceptance of at least the proximal portion of the grasped implantable device.

In some embodiments, the elongated member can be a wire. The distal end of the elongated member may also be shaped to form the grasper. The elongated member is capable of slidable and/or rotational movement within the inner tube. In some instances, the proximal end of the elongated member may be coupled to an actuator, facilitating its movement within the inner tube.

The inner tube may be composed of flexible material such as plastic. The flexible material permits the inner tube to flexurally adapt to the shape of a working channel of an endoscope, for example.

In some embodiments, the grasper is coupled to a grasper locking mechanism. The grasper locking mechanism locks in place the elongated member coupled to the grasper when the grasper has pulled the drawstring of the implantable device and the implantable device has thus been radially collapsed. The grasper locking mechanism thus prevents inadvertent release of the collapsed implantable device.

In one embodiment, the repositioning device includes a retrieval locking mechanism. Once the retrieval hood is advanced distally to capture the collapsed implantable device, the inner tube with the elongated member disposed therein is secured with respect to the endoscope and therefore also the retrieval hood, thus preventing release of the collapsed implantable device with its collapsed barbs from the retrieval hood during the movement or removal. This minimizes the risk of damage to surrounding tissue.

In some embodiments, the repositioning procedure can be viewed and/or guided with a fluoroscope. A distal end of the inner tube may be marked with a radiopaque marker thus facilitating viewing and positioning of the inner tube with respect to the implantable device. Other features of the repositioning device and/or the implantable device may be marked with radiopaque markers thus facilitating the viewing and/or positioning of the features in order to sufficiently collapse the implantable device.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG. 1 shows an exemplary embodiment of a repositioning device;
FIGS. 2A-2F are a series of schematic diagrams showing an exemplary embodiment of the invention capturing a proximal portion of an implantable device for repositioning;
FIGS. 3A-3F are another series of schematic diagrams showing an exemplary embodiment of the invention retrieving an implantable device in the intestine;
FIGS. 4A-4B show an alternative embodiment of the invention using a rotary actuator;
FIG. 5 shows an alternative embodiment using a rat-tooth grasper;
FIGS. 6A-6C show an alternative embodiment of a retrieval hood; and
FIGS. 7A and 7B show an endoscope used to view the repositioning process.

### DETAILED DESCRIPTION OF THE INVENTION

A description of preferred embodiments of the invention follows.

Gastrointestinal implants can be used for a number of treatments, at least some of which are described in U.S. Patent Application Serial No. 10/339,786, filed on January 9, 2003 published as US-A-2004/07004. Implants placed within the gastrointestinal tract are typically subject to substantial mechanical forces related to the digestion process. For example, an implant placed within the intestine, distal to the pyloric sphincter, will be subjected to peristaltic forces tending to push and pull the implant along the intestine. To keep the implant in place, an anchoring device is required. Anchoring can include conventional surgical techniques, such as sutures, staples, surgical adhesives, etc. Anchoring within the intestine, however, poses a unique set of challenges. At least some anchoring devices use an interference fit, placing an implant device having a relaxed diameter larger than the diameter offered by the intestine. Other anchoring devices use barbs that are adapted to penetrate into the surrounding muscular tissue of the gastrointestinal tract. Examples of anchors used for anchoring implants are described in U.S. Patent Application No. 10/858,852 filed on June 1, 2004, published as US-A-2005/25 020.

Anchors relying on interference fit, barbs, or a combination of both typically have relaxed dimensions greater than the diameter of the intestine (e.g., greater than twenty five millimeters in an adult human). For example, the implant may be delivered to the intended location in a compressed state using a catheter having an internal diameter of only about 12 millimeters. When the implant is deployed within the intestine it expands to its implanted size. For example, to place an implant into the proximal duodenum, a catheter can be inserted through the patient's nose or mouth, through the esophagus, stomach and pyloric sphincter. The implanted devices can be compressed again prior to and/or during repositioning or removal. With this in mind, some implants include a means to facilitate compression, such as a drawstring. Examples of implants having drawstrings are described in U.S. Patent Application Serial No. 10/858,851, filed on June 1, 2004, published as US-A-2005 125 075.

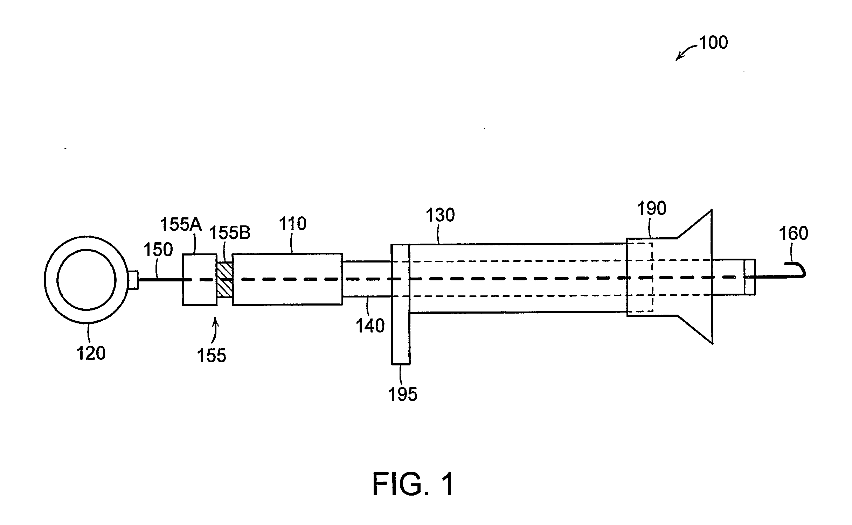

FIG. 1 is a representative view of one embodiment of a repositioning device 100. The repositioning device 100 may include a handle 110 supporting an actuator 120. The repositioning device 100 further may include an elongated member 150, such as a wire. The elongated member 150 is slidably disposed within the handle 110. The actuator 120 is adapted to attach to a proximal end of the elongated member 150. The repositioning device 100 further may include an inner tube 140. The inner tube 140 defines a lumen within which the elongated member 150 is slidably disposed. The inner tube 140 is adapted for insertion into a natural bodily lumen through an endoscope working channel or a catheter. The inner tube 140 is fixed to a distal end of the handle 110.

A grasper 160, a hook in this embodiment, is coupled at a distal end of the elongated member 150 and is adapted to grasp a feature of an implantable device. For example, a drawstring is provided on some implantable devices such that manipulation of the drawstring can reduce at least one dimension (e.g., the diameter) of the implantable device.

The elongated member 150 slidably fits through a hole within the handle 110, and is attached to the actuator 120. The actuator 120 and the handle 110 may be operated manually from a site external to a body. For example, the handle 110 and the actuator 120 can be used to maneuver the elongated member 150 and grasper 160 disposed at the distal end of the elongated member 150. The handle 110 may also be manually manipulated to maneuver the inner tube 140.

The elongated member 150 may be several feet in length. Preferably, the elongated member 150 is formed of a flexible material to facilitate navigation through a medical instrument, for example, through the working channel of an endoscope within a natural bodily lumen. Further, the elongated member 150 should be composed of a biocompatible material. Such materials may include polymers and certain metals, such as Nitinol or stainless steel. The elongated member 150 is coupled at its distal end to the grasper 160.

In some embodiments, the grasper 160 may be a hook. The grasper 160 is attached to the distal end of the elongated member 150. The grasper 160 may be any means of grasping a drawstring of an implantable device. The grasper 160 may be attached to the elongated member 150 by various mechanical, chemical, welding or bonding means. The grasper 160 may be formed of a biocompatible material such as polymers and metals such as Nitinol or stainless steel. In one embodiment, the distal end of the elongated member 150 is shaped to form a hook.

The grasper 160 attached to a distal portion of the elongated member 150, is disposed within a lumen of the inner tube 140. The inner tube 140 may be several feet in length in order to extend from a proximal portion of an implantable device to outside of a body. The dimensions of the inner tube may be such that it adapts to the working channel of an endoscope. The inner tube 140 may be made of a biocompatible and flexible material such as certain polymers. Such polymers may include silicone, polyurethane, polyethylene and certain low friction fluoropolymer materials such as PTFE, PFA or FEP.

In one embodiment, the grasper 160 is coupled to a grasper locking mechanism 155 through the elongated member 150. The grasper locking mechanism 155 is disposed at a proximal portion of the elongated member 150. The grasper locking mechanism 155 locks in place the elongated member 150 coupled to the grasper 160, when the grasper 160 has pulled the drawstring of the implantable device, and the implantable device has thus been radially collapsed. In one embodiment, the grasper locking mechanism 155 is a compression-type locking mechanism. The grasper locking mechanism 155 includes a member 155A, which is threaded onto member 155B. Member 155B is adapted to be fixed within a proximal opening of the handle 110. The elongated member 160 is slidably disposed through the grasper locking mechanism 155, when the grasper locking mechanism 155 is left unlocked. When the grasper 160 has grasped the collapsed implantable device, the grasper locking mechanism 155 may be locked, thus tightening around the elongated member 150 so that the elongated member 160 is fixed and is no longer slidable within the inner tube 140. In other embodiments, the grasper 160 coupled to the elongated member 150 may be locked using other locking mechanisms such as other types of compression locks, screw-type locks, pincher type locks, clamp type locks or any means capable of locking the grasper 160 coupled to the elongated member 150 in place. Exemplary locking devices and methods of using locking devices are described in U.S. Patent Application titled "Endoscope Accessory," attorney docket number 3588.1017-001, filed on even date.

In one embodiment, the actuator 120 may be manually operated by maneuvering the actuator 120 from a site external to a body. The actuator 120 may include one or more features adapted for manual manipulation. For example, the actuator may include one or more looped elements adapted to be operated by fingers and/or thumb. The actuator 120 may advance the elongated member 150 distally by pushing on the actuator 120 by grasping the looped element and pushing it. The actuator 120 may be used to proximally draw the elongated member 150 by grasping and pulling of the looped element. In other embodiments, the actuator may be any means capable of advancing distally or pulling proximally the elongated member 150 coupled to the grasper 160.

The repositioning device 100 may further include an outer tube 130. The outer tube 130 also defines a lumen within which the inner tube 140 may be slidably disposed. In one embodiment, the outer tube 130 is an insertion tube of an endoscope. For example, if the repositioning device 100 is being used within the gastrointestinal tract, the endoscope may be a gastroscope, such as the Olympus GID Q160, 9.8 mm OD. The endoscope may permit the operator to view the removal or repositioning process of the implantable device and to manipulate the relevant features of both the repositioning device 100 and the implantable device during the removal or repositioning process. The positioning and movement of the endoscope may be accomplished by manually maneuvering the proximal end of the endoscope from a site external to the body.

Alternatively, the outer tube 130 may be a separate tube from an endoscope, wherein an endoscope may be place adjacent to the repositioning device 100 in order to view and manipulate the repositioning and/or removal process of the implantable device. The positioning of the outer tube 130 may be accomplished from a site external to the body. The positioning of the outer tube 130 may be manual, for example, by an operator maneuvering a proximal end of the outer tube 130.

In some embodiments, the repositioning device 100 may also include a retrieval hood 190. The retrieval hood 190 may be attached to a distal end of the outer tube 130. The retrieval hood 190 is adapted to capture at least a proximal portion of the implantable device. In some embodiments, the retrieval hood 190 is coupled to the outer tube 130 using an interference fit, where the diameter of the proximal end of the retrieval hood 190 is slightly larger than the distal end of the outer tube 130. In other embodiments, the retrieval hood 190 may be coupled to the outer tube 130 using alternative mechanical, chemical, or bonding techniques.

The retrieval hood 190 may generally be conical in shape. The retrieval hood 190 has openings at both a proximal end and a distal end. As shown, the distal end of the retrieval hood 190 is flared to facilitate capture of an implantable device to be repositioned. In some embodiments, the retrieval hood 190 is made of a flexible material to facilitate its atraumatic placement within a body and to better accommodate at least the proximal portion of the implantable device prior to repositioning. The retrieval hood 190 may be made of a transparent, biocompatible rigid plastic such as polycarbonate or a flexible polymer such as polyurethane, PVC or silicone.

The additional visibility offered by the transparent retrieval hood 190 may be beneficial to the repositioning procedure. For example, if the repositioning device 100 is used through the working channel of an endoscope, (when the endoscope is the outer tube 130) the transparent retrieval hood 190 may allow for a wide field of view. Alternatively, a transparent retrieval hood 190 may also allow for easier viewing from an endoscope external to the repositioning device 100.

The repositioning device 100 may include a retrieval locking mechanism 195. In one embodiment, the retrieval locking mechanism 195 is a pincher-type lock. The retrieval locking mechanism 195, which is slideable upon the inner tube 140 is positioned at the proximal end of the outer tube 130, on the inner tube 140. Once the retrieval hood 190 is advanced over the implantable device to capture it, the pincher-type retrieval locking mechanism 195 is then pinched on the inner tube 140. The inner tube 140 with the elongated member 150 disposed therein is thus locked into place with respect to the outer tube 130 and the retrieval hood 190. This prevents inadvertent release of the radially-collapsed implantable device. In other embodiments, the inner tube 140 and elongated member 150 may be locked with respect to the retrieval hood 190 using other locking mechanisms such as compression locks, other screw-type locks, pincher-type locks, clamp-type locks or any means capable of locking the inner tube 140 and elongated member 150 in place.

The retrieval locking mechanism 195 is beneficial in preventing damage to surrounding tissue when the implantable device is removed or repositioned in the natural bodily lumen. If the inner tube 140 and elongated member 150 are not locked with respect to the retrieval hood 190, the implantable device captured within the retrieval hood 190 may release, thereby moving distal to the retrieval hood 190 allowing it to expand and exposing anchoring barbs to the tissues. Thus, when the implantable device is removed or repositioned within the natural bodily lumen, the exposed and expanded implantable device would be dragged within the natural bodily lumen, resulting in possible tissue damage.

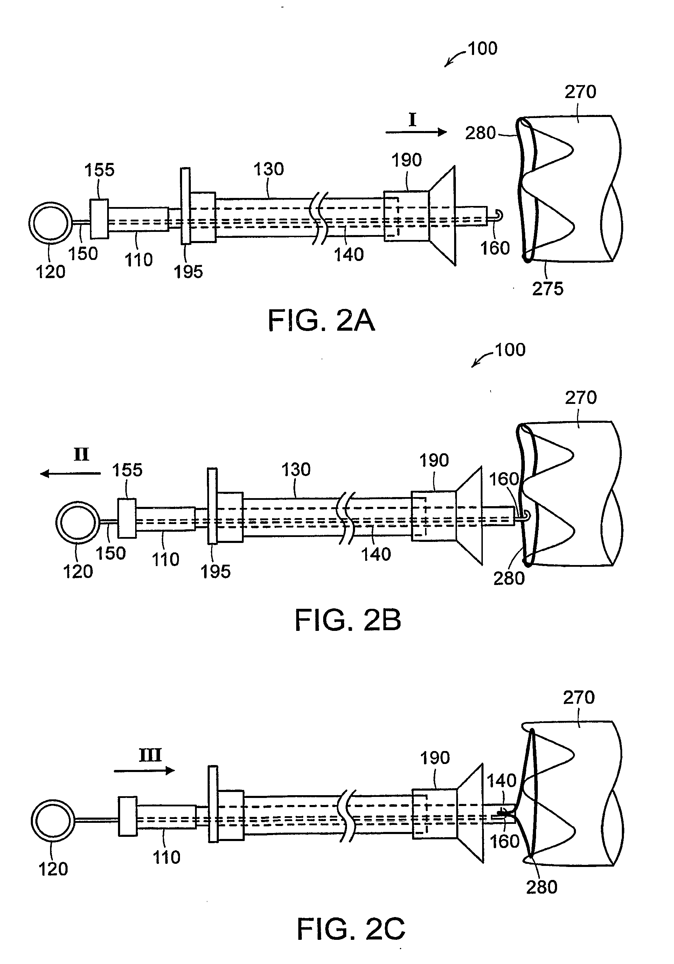

A method of using the repositioning device 100 to capture at least a proximal portion of an implantable device 270 for repositioning and removal is shown in FIGS. 2A-2F. As shown in FIG. 2A, the grasper 160 coupled to the distal end of the elongated member 150, is advanced towards a drawstring 280 of the implantable device 270 by pushing on the actuator 120 (as indicated by arrow I.) The distal end of the grasper 160 can extend distally beyond the outer tube 130, the retrieval hood 190, and the inner tube 140.

As shown in FIG. 2B, the grasper 160 extending distally beyond the inner tube 140, engages a portion of the drawstring 280 of the implantable device 270. The actuator 120 is then used to proximally draw the grasper 160 and the engaged portion of the drawstring 280 (as indicated by arrow II..)

As shown in FIG. 2C, the grasper 160 and the engaged portion of the drawstring 280 are drawn proximally into the distal end of the inner tube 140, reducing slack in the drawstring 280. The inner tube 140 with the grasper 160 and the engaged drawstring 280 disposed in it distal end, is then advanced distally (indicated by the direction of arrow III.) The distal advancement of the inner tube 140 may be accomplished by manipulating the handle 110 coupled to the inner tube 140.

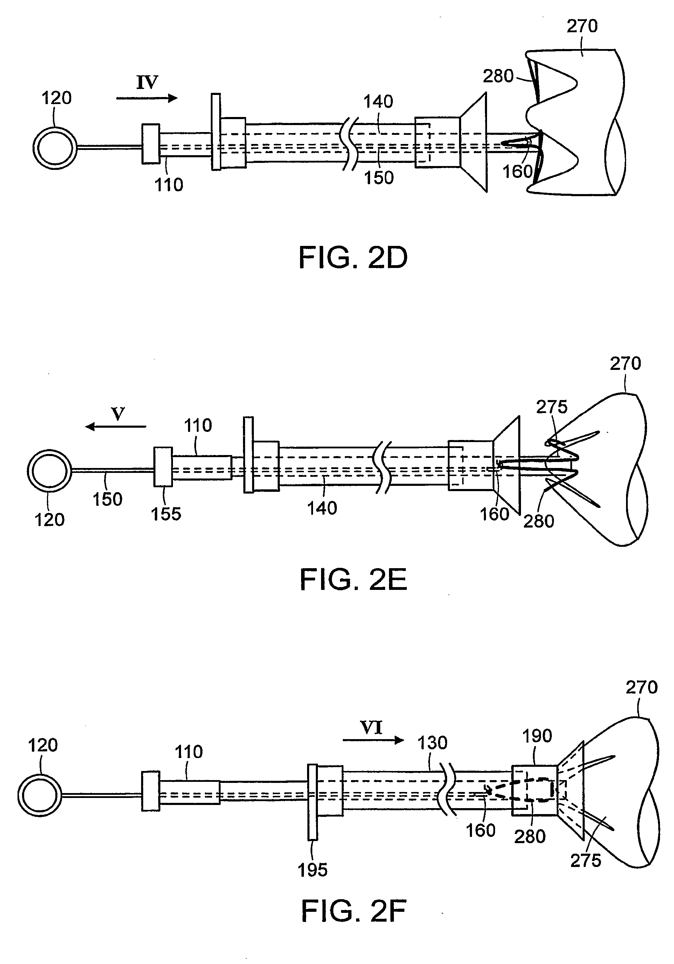

As shown in FIG. 2D, the inner tube 140 is advanced distally until it is within an interior portion of the implantable device 270, or beyond the proximal plane of the implantable device 270 (as indicated by arrow IV.)

As shown in FIG. 2E, once the inner tube 140 is positioned within the interior of the implantable device 270, the actuator 120 is proximally pulled so that the grasper 160 coupled to the elongated member 150 pulls the engaged drawstring 280 proximally into the inner tube 140 (as indicated by arrow V.) When the engaged drawstring 280 is pulled by the grasper 160, the engaged drawstring 280, is also drawn within the lumen of the inner tube 140 sufficiently to radially collapse the implantable device 270, thereby detaching it from the surrounding anatomy. For example, some implants include an anchor or stent having barbs 275 adapted to pierce the surrounding muscular tissue of the intestine. As the drawstring 280 is withdrawn, the anchor or stent is collapsed radially until the barbs 275 are dislodged from the surrounding tissue. At least a proximal portion of the implantable device 270, is therefore radially collapsed.

The positioning of the inner tube 140 coupled to the grasper 160 within the interior of the implantable device 270, is advantageous in preventing damage to surrounding tissue within the natural bodily lumen. As the engaged drawstring 280 is pulled proximally into the inner tube 140, the implantable device 270 is radially collapsed, therefore avoiding significant axial pull on the drawstring 280. This avoids unnecessary dragging of the implantable device 270 through the natural bodily lumen, thus decreasing the chances of tissue damage cause by the exposed barbs 275.

Once the implantable device 270 has been sufficiently radially collapsed by the grasper 160, the elongated member 150 is locked into place by the grasper locking mechanism 155. The elongated member 150 is thus, no longer slidable within the inner tube 140 and the handle 110, but is fixed. The elongated member 150 remains fixed until the grasper locking mechanism 155 is unlocked.

As shown in FIG. 2F, once the implantable device 270 is sufficiently collapsed and locked into place by the grasper locking mechanism 155, the outer tube 130 coupled to the retrieval hood 190 is advanced distally over the inner tube 140 and the radially-collapsed implantable device 270 (as indicated by arrow VI). As the retrieval hood 190 is advanced, it preferably captures at least a proximal portion of the implantable device 270. If the outer tube 130 is an insertion tube of an endoscope, the proximal portion of the endoscope may be manually maneuvered from a site outside of the body in order to centralize the collapsed implantable device 270 within the flared distal end of the retrieval hood 190. Similarly, if the outer tube 130 is a tube distinct from an endoscope, such as a catheter, the proximal end of the outer tube 130 may be maneuvered manually and/or from a site external to the body.

Advancing the retrieval hood 190 over the implantable device 270 may be advantageous in avoiding damage to surrounding tissue. Because the retrieval hood 290 is being advanced over the implantable device 270, at least proximally facing collapsed barbs 275 are covered and will not traumatize the tissue within the natural bodily lumen. The distal facing barbs 275, even if left uncovered will not penetrate into the tissue as they are facing opposite to the direction of withdrawal (indicated by arrow V.) and therefore will not cause damage to surrounding tissue. This facilitates the safe removal or repositioning of the implantable device 270 within the natural bodily lumen.

Once the retrieval hood 190 adequately captures the collapsed implantable device 270, the inner tube 140 and elongated member 150 are locked with respect to the retrieval hood 190 using the retrieval locking mechanism 195, thereby preventing the inadvertent release the implantable device 270 and thereby exposing barbs 275. Once captured and locked into place, the repositioning device 100 and the implantable device 270 can be safely removed from the body or repositioned within the natural bodily lumen as one unit.

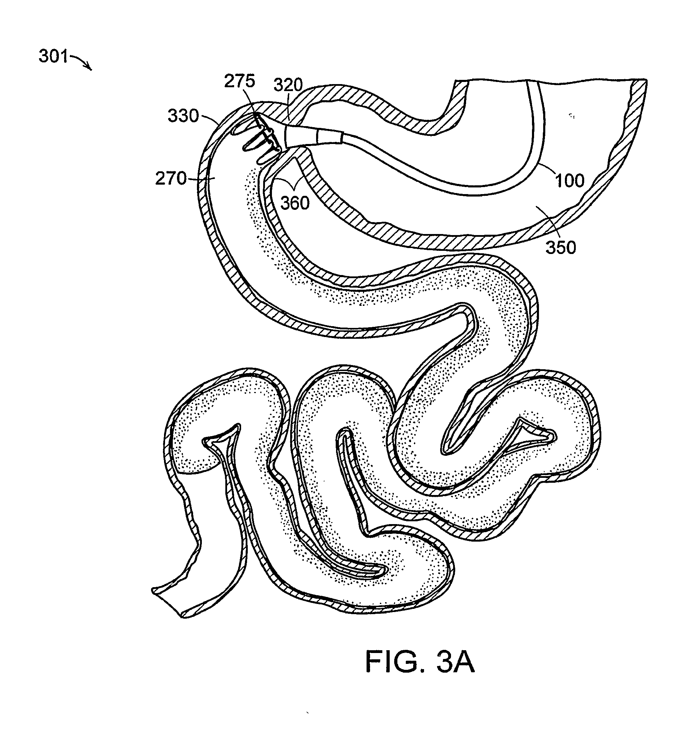

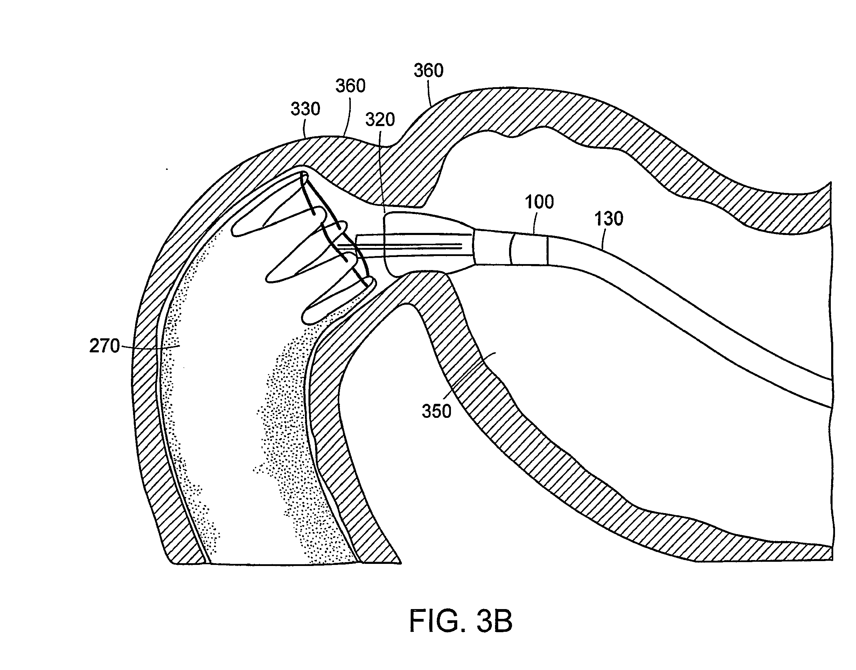

Another illustration of the removal process is presented in FIGS. 3A-3F for an application within a gastrointestinal tract 301. The implantable device 270 is secured or attached in the pyloric region 360 of the stomach 350 or, as shown in FIG. 3A, just distal to the pylorus 320 in the proximal portion of the duodenum 330. As shown in FIG. 3B, the repositioning device 100 is advanced distally from the outside of a body through the esophagus (not shown) and further through the stomach 350 and the pyloric region 360 of the stomach 350 in order to reach the proximal portion of the implantable device 270. Preferably, a distal portion of the repositioning device 100 is advanced through the pyloric sphincter 320 extending at least partially into the proximal duodenum 330.

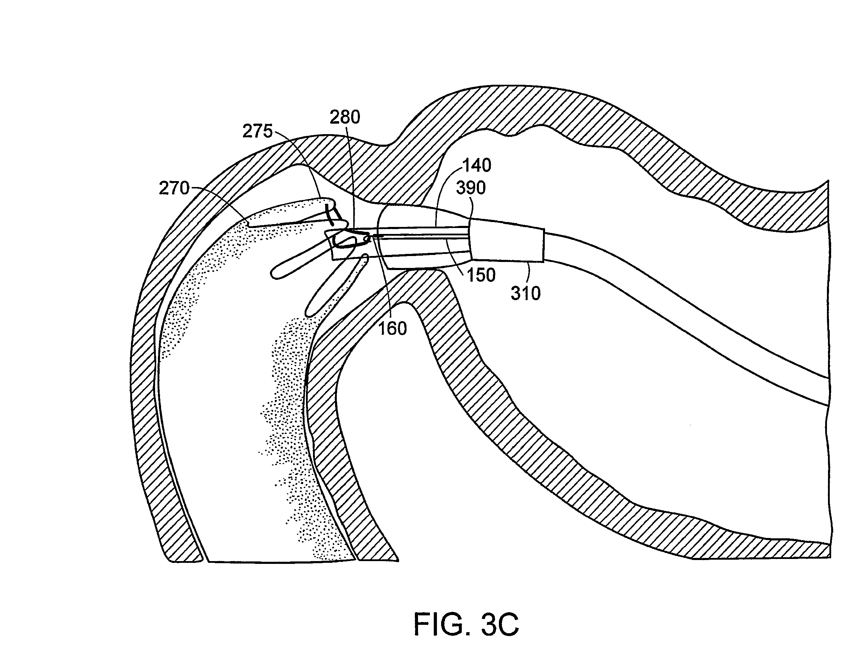

As shown in FIG. 3C, the inner tube 140 with the grasper 160 and the engaged drawstring 280 disposed within the distal end of the inner tube 140, is advanced distally until it is within the interior, or beyond the proximal plane of the implantable device 270. The grasper 160, can then be pulled proximally operating the drawstring 280, thereby radially collapsing a proximal portion of the implantable device 270. The barbs 275 of the implantable device 270 are dislodged from the surrounding tissue. Once the implantable device 270 is sufficiently collapsed, the elongated member 150 coupled to the grasper 160 can be locked into place by locking the grasper locking mechanism (not shown) in order to prevent release of the collapsed implantable device 270.

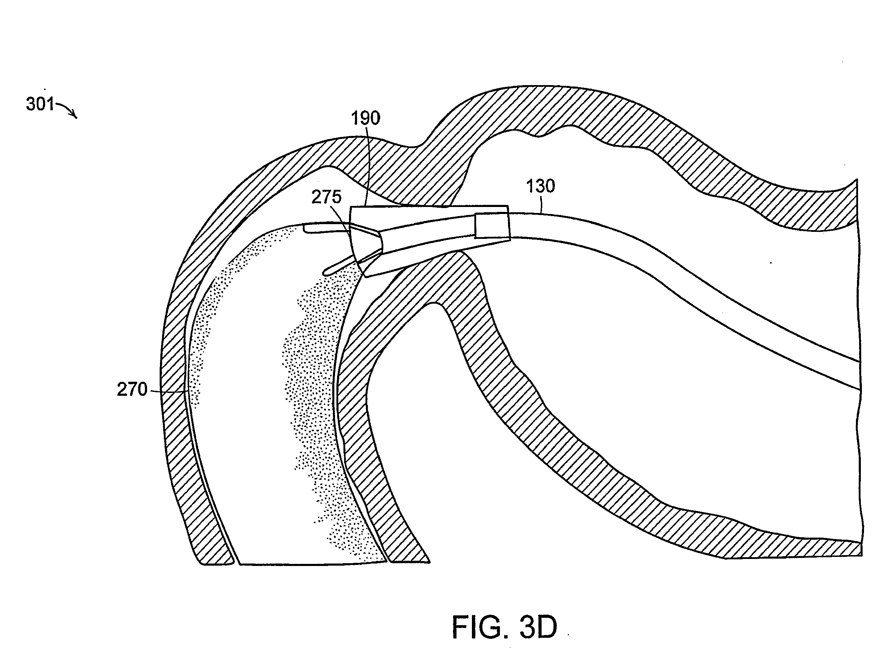

As shown in FIG. 3D, once the implantable device 270 has been radially collapsed, the outer tube 130 coupled to the retrieval hood 190 is advanced distally in order to capture a collapsed proximal portion of the implantable device 270 and the dislodged barbs 275. If the outer tube 130 is the insertion tube of an endoscope, the proximal portion of the endoscope may be maneuvered from a site external to the body in order to center the collapsed implantable device 270 and collapsed barbs 275 within the flared head of the retrieval hood 190. For example, the endoscope may be a gastroscope, such the Olympus GID Q160, 9.8mm OD.

Similarly, if the outer tube 130 is a tube distinct from an endoscope, the proximal portion may be maneuvered to centralize the collapsed implantable device 270 and the dislodged, collapsed barbs 275 within the flared end of the retrieval hood 190. The centralization within the retrieval hood 190, which promotes a complete capture of the proximal end of the collapsed implantable device 270 and the collapsed barbs 275 by the retrieval hood 190, reduces the chances of damage to the surrounding tissue, which may be caused by protruding barbs 275 from the retrieval hood 190, when the implantable device 270 and the repositioning devices 100 are removed from the body by being drawn proximally through the gastrointestinal tract 301 and esophagus.

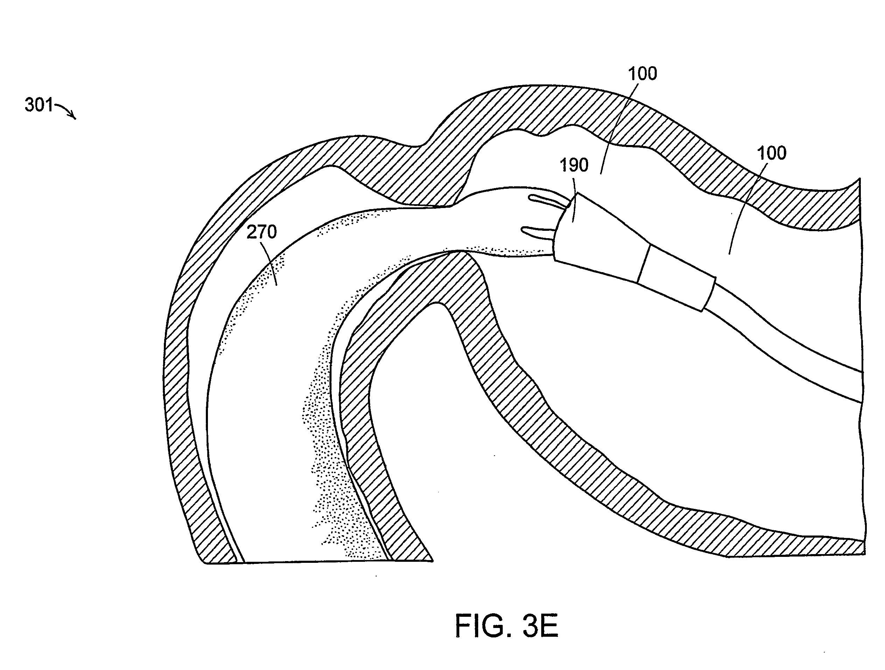

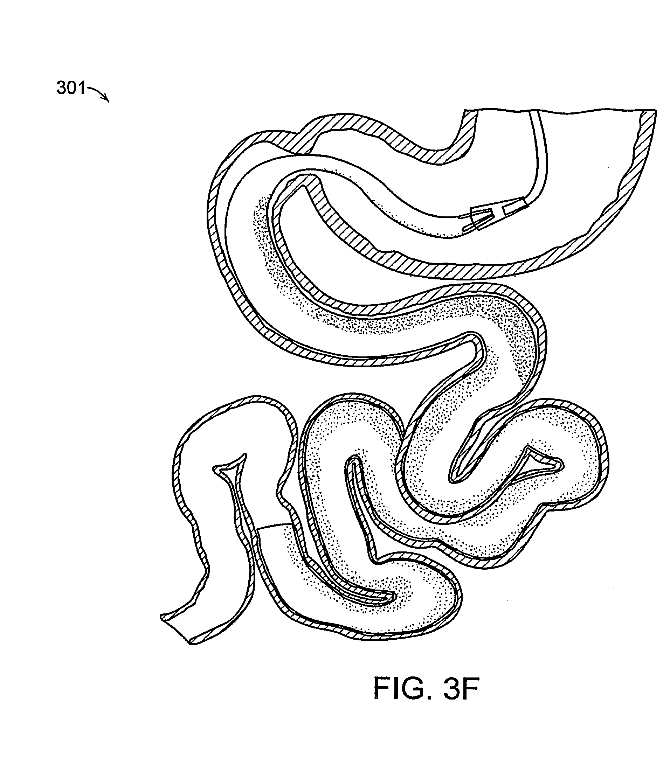

Once effectively captured in the retrieval hood 190 and locked in place by the retrieval locking device, the implantable device 270 and the repositioning device 100 may be repositioned to a different location within the gastrointestinal tract 301 or removed from the body as one unit as shown in FIG. 3E and 3F. When removing an implantable device, this unit is proximally drawn through the esophogus in a safe manner.

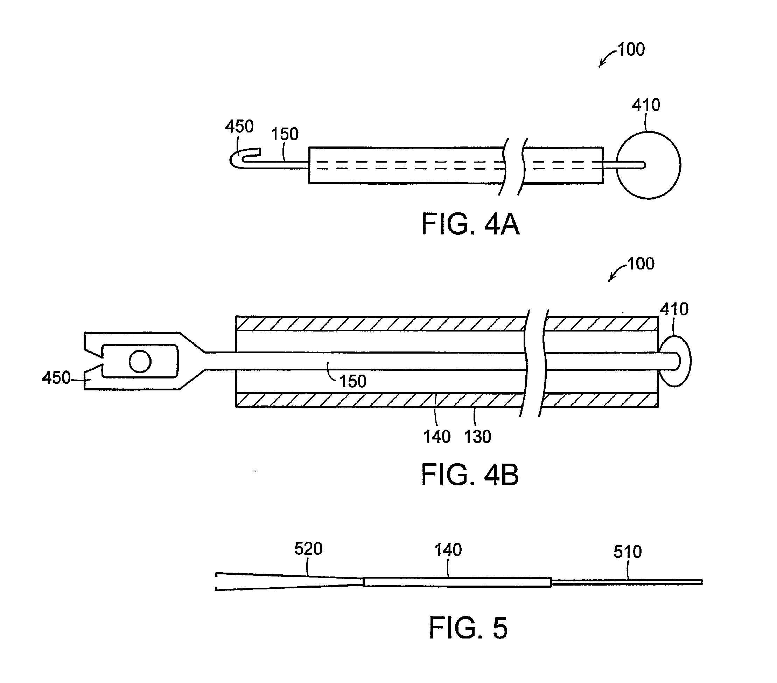

An alternative embodiment is shown in FIGS. 4A and 4B, where the repositioning device 100 includes a rotary actuator 410. The rotary actuator 410 can be used to collapse the implantable device. The rotary-actuated device 100 may similarly include the inner tube 140, the elongated member 150 and a grasper 450. Once the grasper 450 captures a portion of the drawstring of the implantable device, a rotary actuator 410 spins the grasper 450 causing the drawstring to wind about the grasper 450. In one embodiment, the grasper 450 may be a spade with a notch as shown in FIG. 4B. In other embodiments, the grasper 450 may be a hook or any means capable of engaging the drawstring. For example, the distal end of the elongated member 150 can be shaped to form a hook as shown in FIG. 4A.

The winding action causes the drawstring to wrap about the grasper 450, thereby operating the drawstring and radially collapsing the implantable device. Once the implantable device has been radially collapsed the proximal portion of the implantable device can be captured by a retrieval hood when provided as previously described. The entire device 100 and the implantable device may then be removed in a similar manner to that described in FIGS. 3A-3F.

An advantage provided by the rotational device is that it is not stroke-length limited. Stroke-length refers to the length of translation provided by the grasper within the inner tube. This translation may be limited by the physical dimensions of the device and will limit the length of drawstring that can be withdrawn into the sleeve. There is no similar limitation to the amount of rotation (i.e., number of turns). As long as the hook and wire are capable of rotating, the number of rotations can be varied to selectably wind a desired length of the drawstring about the wire.

It may be possible that with a fixed stroke length, if the drawstring on the anchor stretches, the grasper may not be able to fully collapse the anchor. Additionally, much of the force applied at the proximal end of the reciprocating device may be lost through the shaft as the shaft buckles. Almost all of the torque provided at the proximal end of the rotational device can be delivered to its distal end while keeping it flexible. Also, the actuation of the rotational device may provide improved ergonomics, since it is translated separately from its rotational motion. This may make it easier to move the drawstring collapse point proximal or distal to dislodge the anchor or stent, while keeping the drawstring collapsed.

An alternative type of grasper is shown in FIG. 5, where the grasper is a rat-tooth type grasper 510. The rat tooth grasper 510 is advanced within the interior of the drawstring as described in previous figures. The rat tooth grasper 510 is then actuated so that its jaws 520 grasp the drawstring between the two jaws 520. The rat tooth grasper 510 is advantageous in that the drawstring is easily released if desired by simply opening the jaws 520 of the rat tooth grasper 510. The jaws 520 are opened by advancing distally the jaws 520 until they exit the inner tube 140. The jaws 520 are closed by pulling the jaws into the inner tube 140.

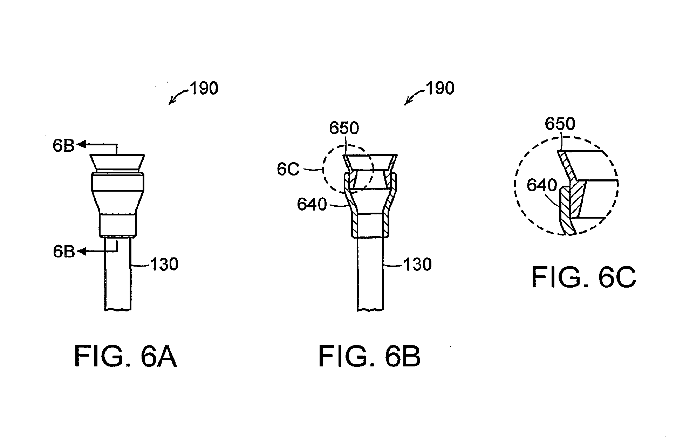

An alternative or additional embodiment of the repositioning device 100 is show in FIG. 6A-6C, wherein the retrieval hood 190 includes a feature adapted to steer the grasper towards the center of the implanted device. As shown in the cross section in 6B and 6C, an interior ramp 640 is provided over at least a portion of the interior of the retrieval hood 190. Additionally, the retrieval hood 190 includes a flared end 650. The proximal end of the retrieval hood 190 may be coupled to the outer tube 130 or alternatively, to the distal end of an insertion tube of an endoscope.

For example, the angle of the flared end 650 can extend over about 10 to 90 degrees about the interior of the retrieval hood 190 as shown in FIG. 6B. The interior ramp 640 is aligned to centrally position the distal end of the inner tube 140 with the grasper 160 and engaged drawstring 280 disposed therein, within the interior of the implantable device prior to and as it is radially collapsing the device. This may be advantageous, because the inner tube 140 and grasper 160 tend to be eccentric, or biased towards one side since the working channel of the endoscope through which the grasper is positioned is eccentric. Distal advancement of the inner tube 140 through the retrieval hood 190 towards the implanted device, allows the inner tube 140 and elongated member 150 to bend towards a central position within the drawstring of the implantable device, and pull the drawstring. This allows for primarily uniform radial force to be applied to the drawstring in order to radially collapse the implantable device.

As the retrieval hood 190 is advanced distally to capture the radially collapsed device, the interior ramp 640 along with the angle of the flared end 650 allows the elongated member along with the radially collapsed implantable device to be centralized within the retrieval hood 190, therefore facilitating the removal or repositioning of the implantable device.

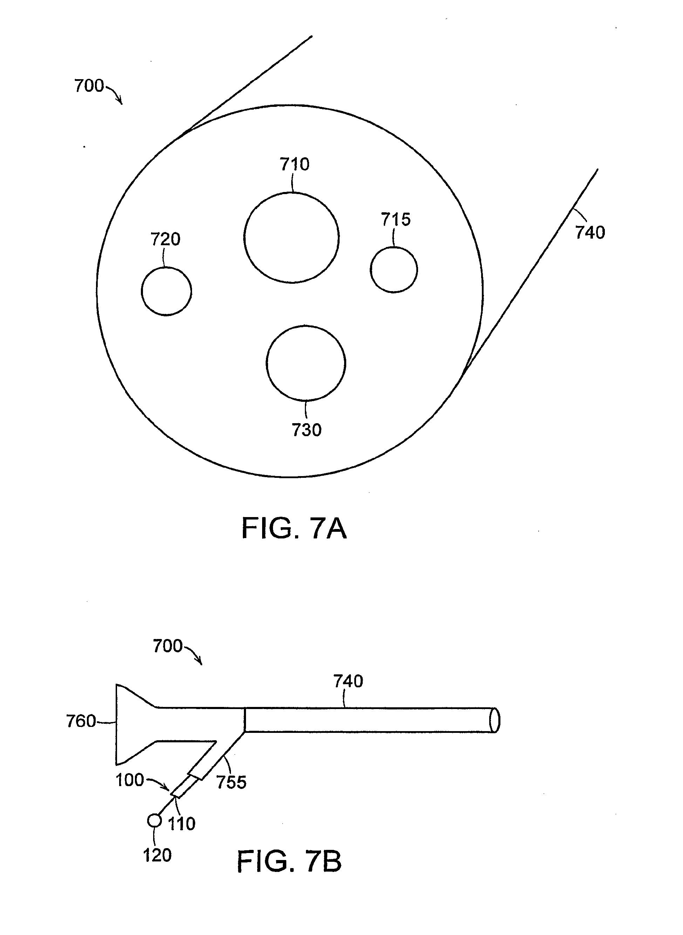

As shown in FIG. 7A and 7B, all procedures just described can be observed by the endoscopist using an endoscope and camera. Such a visual aid will facilitate operation of the proximal controls (e.g., handle 110 and actuator 120) to position the grasper 160 near the drawstring, to engage the drawstring, to position the inner tube 140 within the interior of the implantable device so that the implantable device may be sufficiently collapsed, to confirm that the barbs are sufficiently detached, and to capture the proximal end of the implant with the retrieval hood 190. Beneficially, the retrieval hood 190 can be formed of a transparent material, such as polycarbonate, PVC or polyurethane. Such additional visibility offered by the transparent retrieval hood 190 is advantageous to the removal procedure, by allowing clear viewing of the repositioning procedure.

As shown in FIG. 7A, the distal end of the endoscope 700 includes an objective lens 710, through which the repositioning procedure can be viewed. A light source 720 may be provided to enable brighter viewing of the repositioning procedure. An irrigation port 715 may also provided. Additionally, the distal end of the endoscope 700 may include an instrument channel outlet 730.

As described in previous figures, the outer tube 130 is the insertion tube 740 of the endoscope 700 as shown in FIG. 7B. The distal end of the insertion tube 740 of the endoscope 700 may include the instrument channel outlet 730, as shown in FIG. 7A. The inner tube 140 is slidably disposed within the insertion tube 740 of the endoscope 700, and may be distally advanced or proximally pulled through the instrument channel outlet 730. The proximal end of an instrument channel 755 is shown. The actuator 120 and handle 110 of the repositioning device 100 may be maneuvered from the instrument channel 755, through which the inner tube 140 of the repositioning device 100 is slidably disposed. The procedure may be viewed and directed by an endoscopist, for example, looking through an eyepiece 760 or at an image projected on a monitor.

Alternatively, not according to the invention, the outer tube 130 may be a distinct tube from the insertion tube 740 of the endoscope 700. In this case, if the operator wishes to view the repositioning procedure through the endoscope 700, the endoscope 700 may be positioned adjacent to the repositioning device 100 within the natural bodily lumen. The viewing and/or guiding of the repositioning procedure is facilitated by the transparent retrieval hood 190.

An endoscope may be used in combination with, or independent of a fluoroscope. Alternatively, fluoroscopy may be utilized to guide and view the repositioning procedure independent of endoscopy.

Fluoroscopy may be used to guide the removal or repositioning of an implantable device. The distal end of the inner tube 140 may be marked with a radiopaque marker. Fluoroscopy may be used to confirm that the distal end of the inner tube 140 is positioned within the interior of the implantable device. If the inner tube 140 is not properly positioned, the radiopaque marker facilitates viewing of the distal end of the inner tube 140 and thus adjustment of the inner tube 140 to sufficiently radially collapse the implantable device.

Alternatively, or in addition, a combination of radiopaque markers may be provided on the repositioning device 100 as well as on the implantable device. This may particularly be useful if one wishes to utilize fluoroscopy independent of an endoscope. For example, a portion of the drawstring of the implantable device may be marked with a radiopaque marker. The grasper 160 or elongated member 150 may be marked with a radiopaque marker. In this way, an endoscope may not be required, as the entire repositioning procedure and the relevant parts which need to be guided during the repositioning procedure, are sufficiently displayed on a monitor.

While this invention has been particularly shown and described with references to preferred embodiments thereof, it will be understood by those skilled in the art that various changes in form and detail may be made therein without departing from the scope of the invention encompassed by the appended claims.

## Claims

1. A device for repositioning an implantable device (270) within a natural bodily lumen comprising:
an endoscope providing an outer tube (130) defining a lumen;
an inner member (140) adapted for insertion into the outer tube (130);
a grasper (160) adapted to engage an implantable device (270); and
a retrieval hood (190), which hood (190) is atraumatic and is attached to and fitting over a distal end of the endoscope;
wherein the endoscope is slidably mounted around the inner member (140) to advance the atraumatic retrieval hood (190) distally to capture at least a proximal portion of the implantable device (270) when the device (270) is collapsed;
**characterised by** the inner member (140) being an inner tube (140) defining a lumen;
by an elongated member (150) having a proximal end and a distal end, the elongated member slidably disposed within the lumen of the inner tube (140);
in that the grasper (160) is disposed at the distal end of the elongated member (150) and is adapted to engage a drawstring (280) of the implantable device (270) to collapse at least a portion of the implantable device when operated;
by the retrieval hood (190) being a flared retrieval hood (190);
in that the grasper (160) is adapted to be drawn proximally into a distal portion of the inner tube (140) when the drawstring (280) is engaged by the grasper (160);
in that the endoscope is slidably mounted around the inner tube (140) to advance the atraumatic flared retrieval hood (190) distally to capture at least a proximal portion of the implantable device (270) when the device (270) is collapsed and the grasper (160) is locked in place relative to the inner tube (140); and
by the repositioning device further comprising the implantable device (270), comprising:
a flexible, floppy sleeve, open at both ends, to extend into the duodenum;
a collapsible anchor coupled to a proximal portion of the sleeve; and
the drawstring (280), threaded through a proximal end of the anchor and drawn into the inner tube by the grasper (160);
at least a proximal portion of the anchor being captured within the retrieval hood (190).

2. The repositioning device of claim 1, wherein the elongated member (150) is a wire.

3. The repositioning device of claim 1, wherein the grasper (160) is a hook.

4. The repositioning device of claim 1, wherein the grasper (160) is a spade.

5. The repositioning device of claim 1, wherein the grasper (160) is a rat-tooth grasper.

6. The repositioning device of claim 1, wherein the inner tube (140) is flexible.

7. The repositioning device of claim 1, wherein the retrieval hood (190) is flexible transparent plastic.

8. The repositioning device of claim 1, wherein the retrieval hood (190) includes an alignment feature to facilitate engagement of the implantable device (270).

9. The repositioning device of claim 8, wherein the alignment feature of the retrieval hood is an interior ramp (640).

10. The repositioning device of claim 1, wherein the elongated member (150) is rotatably disposed within the lumen of the inner tube (140).

11. The repositioning device of claim 1, further comprising a retrieval locking mechanism (195) for locking the grasper (160) with respect to the retrieval hood (190).

12. The repositioning device of claim 1, further comprising a radiopaque marker marking the distal end of the inner tube (140).

13. The repositioning device of claim 1, further comprising a grasper locking mechanism (155) to lock the grasper (160) in place relative to the inner tube (140) once the implantable device (270) is collapsed.

14. The repositioning device of claim 1, wherein the implantable device (270) further comprises barbs extending from the exterior surface of the anchor.

15. The repositioning device of claim 1, wherein the implantable device (270) further comprises protrusions extending from the exterior surface of the anchor.

## Patentansprüche

1. Vorrichtung zum Repositionieren einer implantierbaren Vorrichtung (270) in einem natürlichen Körperlumen, wobei die Vorrichtung Folgendes umfasst:
ein Endoskop, das einen äußeren Schlauch (130) bereitstellt, der ein Lumen definiert;
ein inneres Element (140), das zum Einführen in den äußeren Schlauch (130) eingerichtet ist;
einen Greifer (160), der zum Ineingriffnehmen einer implantierbaren Vorrichtung (270) eingerichtet ist; und
eine Rückholhaube (190), wobei die Haube (190) atraumatisch ist und an einem distalen Ende des Endoskops angebracht ist und über dieses passt;
wobei das Endoskop verschiebbar um das innere Element (140) herum montiert ist, um die atraumatische Rückholhaube (190) distal vorzuschieben, um mindestens einen proximalen Teil der implantierbaren Vorrichtung (270) zu erfassen, wenn die Vorrichtung (270) zusammengeklappt ist;
**dadurch gekennzeichnet, dass** das innere Element (140) ein innerer Schlauch (140) ist, der ein Lumen definiert;
dass ein längliches Element (150) ein proximales Ende und ein distales Ende hat, wobei das längliche Element verschiebbar in dem Lumen des inneren Schlauchs (140) angeordnet ist;
dass der Greifer (160) an dem distalen Ende des länglichen Elements (150) angeordnet ist und dazu eingerichtet ist, einen Zugfaden (280) der implantierbaren Vorrichtung (270) in Eingriff zu nehmen, um mindestens einen Teil der implantierbaren Vorrichtung im Gebrauch zusammenzuklappen;
dass die Rückholhaube (190) eine aufgeweitete Rückholhaube (190) ist;
dass der Greifer (160) dazu eingerichtet ist, proximal in einen distalen Teil des inneren Schlauchs (140) gezogen zu werden, wenn der Zugfaden (280) von dem Greifer (160) in Eingriff genommen wird;
dass das Endoskop verschiebbar um den inneren Schlauch (140) herum montiert ist, um die atraumatische Rückholhaube (190) distal vorzuschieben, um mindestens einen proximalen Teil der implantierbaren Vorrichtung (270) zu erfassen, wenn die Vorrichtung (270) zusammengeklappt ist und der Greifer (160) in Bezug auf den inneren Schlauch (140) eingerastet ist; und
dass die Repositioniervorrichtung weiterhin die implantierbare Vorrichtung (270) umfasst, die Folgendes umfasst:
eine flexible, weiche Hülse, die an beiden Enden offen ist, um sich in den Zwölffingerdarm zu erstrecken;
einen zusammenklappbaren Anker, der mit einem proximalen Teil der Hülse verbunden ist; und
den Zugfaden (280), der durch ein proximales Ende des Ankers gefädelt ist und von dem Greifer (160) in den inneren Schlauch gezogen wird;
wobei mindestens ein proximaler Teil des Ankers in der Rückholhaube (190) erfasst wird.

2. Repositioniervorrichtung nach Anspruch 1, wobei das längliche Element (150) ein Draht ist.

3. Repositioniervorrichtung nach Anspruch 1, wobei der Greifer (160) ein Haken ist.

4. Repositioniervorrichtung nach Anspruch 1, wobei der Greifer (160) ein Spaten ist.

5. Repositioniervorrichtung nach Anspruch 1, wobei der Greifer (160) ein Rattenzahngreifer ist.

6. Repositioniervorrichtung nach Anspruch 1, wobei der innere Schlauch (140) flexibel ist.

7. Repositioniervorrichtung nach Anspruch 1, wobei die Rückholhaube (190) aus flexiblem transparentem Kunststoff ist.

8. Repositioniervorrichtung nach Anspruch 1, wobei die Rückholhaube (190) ein Ausrichtungsmerkmal aufweist, um das Ineingriffnehmen der implantierbaren Vorrichtung (270) zu erleichtern.

9. Repositioniervorrichtung nach Anspruch 8, wobei das Ausrichtungsmerkmal der Rückholhaube eine interne Rampe (640) ist.

10. Repositioniervorrichtung nach Anspruch 1, wobei das längliche Element (150) drehbar in dem Lumen des inneren Schlauchs (140) angeordnet ist.

11. Repositioniervorrichtung nach Anspruch 1, die weiterhin ein Rückholarretiermechanismus (195) zum Arretieren des Greifers (160) in Bezug auf die Rückholhaube (190) umfasst.

12. Repositioniervorrichtung nach Anspruch 1, die weiterhin einen röntgendurchlässigen Marker umfasst, der das distale Ende des inneren Schlauchs (140) markiert.

13. Repositioniervorrichtung nach Anspruch 1, die weiterhin einen Greiferarretiermechanismus (155) umfasst, um den Greifer (160) in Bezug auf den inneren Schlauch (140) an Ort und Stelle zu arretieren, sobald die implantierbare Vorrichtung (270) zusammengeklappt ist.

14. Repositioniervorrichtung nach Anspruch 1, wobei die implantierbare Vorrichtung (270) weiterhin Widerhaken umfasst, die sich von der Außenfläche des Ankers erstrecken.

15. Repositioniervorrichtung nach Anspruch 1, wobei die implantierbare Vorrichtung (270) weiterhin Vorsprünge umfasst, die sich von der Außenfläche des Ankers erstrecken.

## Revendications

1. Un dispositif pour repositionner un dispositif implantable (270) dans une lumière corporelle naturelle, comprenant :
un endoscope fournissant un tube extérieur (130) définissant une lumière ;
un élément intérieur (140) adapté pour s'insérer dans le tube extérieur (130) ;
un dispositif de préhension (160) adapté pour s'engager avec un dispositif implantable (270) ; et
une garniture frontale de récupération (190), laquelle garniture frontale (190) est atraumatique et est attachée à et montée sur une extrémité distale de l'endoscope ;
dans lequel l'endoscope est monté de manière coulissante autour de l'élément intérieur (140) pour faire avancer la garniture frontale de récupération atraumatique (190) distalement pour saisir au moins une partie proximale du dispositif implantable (270) lorsque le dispositif (270) est effondré ;
**caractérisé par** l'élément intérieur (140) étant un tube intérieur (140) définissant une lumière ;
par un élément allongé (150) ayant une extrémité proximale et une extrémité distale, l'élément allongé disposé de manière coulissante dans la lumière du tube intérieur (140) ;
en ce que le dispositif de préhension (160) est disposé à l'extrémité distale de l'élément allongé (150) et adapté pour s'engager avec un cordon de serrage (280) du dispositif implantable (270) pour effondrer au moins une partie du dispositif implantable lorsqu'il est actionné ;
par la garniture frontale de récupération (190) étant une garniture frontale de récupération évasée (190) ;
en ce que le dispositif de préhension (160) est adapté pour être tiré proximalement dans une partie distale du tube intérieur (140) lorsque le cordon de serrage (280) est engagé par le dispositif de préhension (160) ;
en ce que l'endoscope est monté de manière coulissante autour du tube intérieur (140) pour faire avancer la garniture frontale de récupération évasée atraumatique (190) distalement pour saisir au moins une partie proximale du dispositif implantable (270) lorsque le dispositif (270) est effondré et que le dispositif de préhension (160) est verrouillé en place par rapport au tube intérieur (140) ; et
par le dispositif de repositionnement comprenant en outre le dispositif implantable (270) comprenant :
un manchon souple, flexible, ouvert aux deux extrémités, pour s'étendre dans le duodénum ;
un ancrage effondrable accouplé à une partie proximale du manchon ; et
le cordon de serrage (280), enfilé à travers une extrémité proximale de l'ancrage et tiré dans le tube intérieur par le dispositif de préhension (160) ;
au moins une partie proximale de l'ancrage étant saisie dans la garniture frontale de récupération (190).

2. Le dispositif de repositionnement selon la revendication 1, dans lequel l'élément allongé (150) est un fil métallique.

3. Le dispositif de repositionnement selon la revendication 1, dans lequel le dispositif de préhension (160) est un crochet.

4. Le dispositif de repositionnement selon la revendication 1, dans lequel le dispositif de préhension (160) est une bêche.

5. Le dispositif de repositionnement selon la revendication 1, dans lequel le dispositif de préhension (160) est un dispositif de préhension à dents de rat.

6. Le dispositif de repositionnement selon la revendication 1, dans lequel le tube intérieur (140) est flexible.

7. Le dispositif de repositionnement selon la revendication 1, dans lequel la garniture frontale de récupération (190) est en plastique transparent flexible.

8. Le dispositif de repositionnement selon la revendication 1, dans lequel la garniture frontale de récupération (190) inclut un accessoire d'alignement pour faciliter l'engagement du dispositif implantable (270).

9. Le dispositif de repositionnement selon la revendication 8, dans lequel l'accessoire d'alignement de la garniture frontale de récupération est une rampe intérieure (640).

10. Le dispositif de repositionnement selon la revendication 1, dans lequel l'élément allongé (150) est disposé de manière rotative dans la lumière du tube intérieur (140).

11. Le dispositif de repositionnement selon la revendication 1, comprenant en outre un mécanisme de verrouillage de récupération (195) pour verrouiller le dispositif de préhension (160) par rapport à la garniture frontale de récupération (190).

12. Le dispositif de repositionnement selon la revendication 1, comprenant en outre un marqueur radiopaque marquant l'extrémité distale du tube intérieur (140).

13. Le dispositif de repositionnement selon la revendication 1, comprenant en outre un mécanisme de verrouillage du dispositif de préhension (155) pour verrouiller le dispositif de préhension (160) en place par rapport au tube intérieur (140) après que le dispositif implantable (270) est effondré.

14. Le dispositif de repositionnement selon la revendication 1, dans lequel le dispositif implantable (270) comprend en outre des pointes s'étendant depuis la surface extérieure de l'ancrage.

15. Le dispositif de repositionnement selon la revendication 1, dans lequel le dispositif implantable (270) comprend en outre des saillies s'étendant depuis la surface extérieure de l'ancrage.
